# EUROPEAN PATENT SPECIFICATION

(11) **EP 0 355 794 B1**
(45) Date of publication and mention of the grant of the patent: **19.01.1994**
(21) Application number: 89115470.0
(22) Date of filing: 22.08.1989
(51) Int. Cl.: C07K 7/02, C07K 7/10, A61K 37/02

(54) **Neuropeptide Y antagonists**
Antagonisten von Neuropeptid Y
Antagonistes du neuropeptide Y

(30) Priority: 26.08.1988 US 237599
(43) Date of publication of application: 28.02.1990
(73) Proprietor: MERRELL DOW PHARMACEUTICALS INC., Cincinnati Ohio 45215-6300 (US)
(72) Inventor: Krstenansky, John L., Cincinnati Ohio 45208 (US)
(74) Representative: VOSSIUS & PARTNER

(56) References cited:
- No relevant documents have been found.

## Description

This invention relates to novel peptide derivatives which are antagonists of neuropeptide Y.

Porcine neuropentide Y (pNPY) is a 36 amino acid residue peptide that belongs to a unique family of peptides having a wide distribution throughtout the central and peripheral nervous systems. Receptors for NPY are found in the central nervous system and in the periphery. In the brain, NPY is a potent stimulator of food intake, stimulates luteinizing hormone, growth hormone and prolactin, and produces cardiovascular depression. NPY is also a potent peripheral vasoconstrictor and has been reported to cause transient myocardial ischaemia is patients with angina pectoris. Antagonism of these effects is expected to result in reduced blood pressure and accordingly antagonists of NPY are expected to be useful in the treatment of hypertension, vasospasm, and angina, and useful for the suppression of appetite. NPY has been shown to be a potent bronchoconstrictor and has been implicated in the regulation of the resting tone of airways. NPY antagonists would block the NPY induced constriction and result in bronchodilation.

Novel peptide derivatives of formula 1 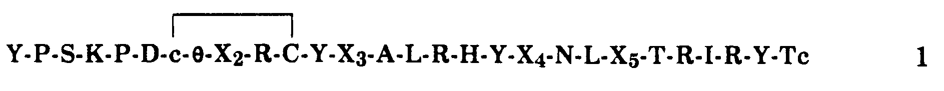
wherein
X₂ is S or A;
X₃ is S or A;
X₄ is L, I, M, Nle, or V;
X₅ is L, I, M, Nle, or V;
Tc is OR' or NHR';
wherein R' is a hydrogen or a (C₁-C₄)alkyl group;
ϑ is a group of the structural formula

-NH-(CH₂)ₙ-CO₂-;

wherein n is an integer of from 1-11 and the pharmaceutically acceptable salts thereof are antagonists of neuropeptide Y. These peptide derivatives inhibit NPY-induced contractions of mouse spleen.

The following common abbreviations of the amino acids and amino and carboxy terminal groups are used throughout this specification:
Ala (or A) - alanine
Val (or V) - valine
Leu (or L) - leucine
Ile (or I) - isoleucine
Pro (or P) - proline
Met (or M) - methionine
Ser (or S) - serine
Thr (or T) - threonine
Cys (or C) - cysteine
cys (or c) - D-cysteine
Tyr (or Y) - tyrosine
Asn (or N) - asparagine
Asp (or D) - aspartic acid
Lys (or K) - lysine
Arg (or R) - arginine
His (or H) - histidine
Nle - norleucine
Aoc - 8-aminooctanoic acid
# - -NH₂
An alkyl group is taken to include straight, branched, or cyclic alkyl groups, for example, methyl, ethyl, propyl, isopropyl, butyl, isobutyl, tert-butyl, pentyl, isopentyl, sec-pentyl, cyclopentyl, hexyl, isohexyl, cyclohexyl and cyclopentylmethyl. An acyl group of from 2 to 10 carbon atoms is taken to include straight, branched, cyclic, saturated and unsaturated acyl groups having 1 or 2 carbonyl moieties per group, for example, acetyl, benzoyl, succinyl, maleyl, and glutaryl. Those peptides wherein the amino group of the amino terminal amino acid is substituted with two alkyl or acyl groups are also considered to be within the scope of the peptides of this invention.

The natural amino acids, with the exception of glycine, contain a chiral carbon atom. Unless otherwise specifically indicated, the optically active amino acids, referred to herein, are of the L-configuration.

The polypeptides of formula 1 can form pharmaceutically acceptable salts with any non-toxic, organic or inorganic acid. Illustrative inorganic acids which form suitable salts include hydrochloric, hydrobromic, sulphuric and phosphoric acid and acid metal salts such as sodium monohydrogen orthophosphate and potassium hydrogen sulfate. Illustrative organic acids which form suitable salts include the mono, di and tricarboxylic acids. Illustrative of such acids are, for example, acetic, glycolic, lactic, pyruvic, malonic, succinic, glutaric, fumaric, malic, tartaric, citric, ascorbic, maleic, hydroxymaleic, benzoic, hydroxybenzoic, phenylacetic, cinnamic, salicylic, 2-phenoxybenzoic and sulfonic acids such as methane sulfonic acid and 2-hydroxyethane sulfonic acid. Salts of the carboxy terminal amino acid moiety include the non-toxic carboxylic acid salts formed with any suitable inorganic or organic bases. Illustratively, these salts include those of alkali metals, as for example, sodium and potassium; alkaline earth metals, such as calcium and magnesium; light metals of Group IIIA including aluminum; and organic primary, secondary and tertiary amines, as for example, trialkylamines, including triethylamine, procaine, dibenzylamine, 1-ethenamine, N,N'-dibenzylethylenediamine, dihydroabietylamine, N-(lower)alkylpiperidine, and any other suitable amine.

As with any generic group of chemical compounds, certain groups are preferred. Applicants prefer those peptide derivatives of formula 1 wherein X₂ is alanine (A). Applicants also prefer those peptide derivatives of formula 1 wherein X₃ is serine (S), as well as those peptide derivatives of formula 1 wherein X₄ or X₅ is isoleucine (I), wherein Tc = NH₂ and wherein ϑ is Aoc. The most preferred peptide derivative of formula 1 is the peptide derivative of formula 2. 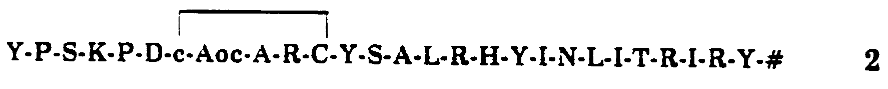
The proteins of this invention can be prepared by a variety of procedures readily known to those skilled in the art. Such procedures include the solid phase sequential procedure which can be performed using established automated methods such as by use of an automated peptide sythesizer.

The resin support employed can be any suitable resin conventionally employed in the art for the solid phase preparation of polypeptides, preferably polystyrene which has been cross-linked with from 0.5 to about 3 percent divinyl benzene, which has been either converted to the p-methylbenzhydrylamine or benzhydrylamine derivative (for C-terminal amides) or chloromethylated or hydroxymethylated to provide sites for ester formation with the initially introduced α-amino protected amino acid (for producing C-terminal alkylamides) and esters.

An example of a hydroxymethyl resin is described by Bodanszky, et al., Chem. Ind. (London) 38, 1597-98 (1966). A chloromethylated resin is commercially available from Bio Rad Laboratories, Richmond, California, and the preparation of such a resin is described by Stewart et al., "Solid Phase Peptide Synthesis" (Freeman & Co., San Francisco 1969), Chapter 1, pp. 1-6. The protected amino acid can be bound to the resin by the procedure of Gisin, Helv. Chem Acta, 56, 1476 (1973). Many resin bound, protected amino acids are commercially available. As an example, to prepare a polypeptide of this invention wherein the carboxy terminal end is a Thr residue, a tert-butyloxycarbonyl (Boc) protected Thr bound to a benzylated, hydroxymethylated phenylacetamidomethyl (PAM) resin can be used and is commercially available.

Following the coupling of the α-amino protected amino acid to the resin support, the protecting group is removed using any suitable procedure such as by using trifluoroacetic acid in methylene chloride, trifluoroacetic acid alone, or HCl in dioxane. The deprotection is carried out at a temperature of between 0°C and room temperature. Other standard cleaving reagents and conditions for removal of specific α-amino protecting groups may be used. After removal of the α-amino protecting group the other amino protected amino acids are coupled step-wise in the desired order. Alternatively, multiple amino acid groups may be coupled by the solution method prior to coupling with the resin supported amino acid sequence.

The α-amino protecting group employed with each amino acid introduced into the polypeptide sequence may be any such protecting group known to the art. Among the classes of α-amino protecting groups contemplated are (1) acyl type protecting groups such as: formyl, trifluoroacetyl, phthalyl, toluenesulfonyl (tosyl), benzenesulfonyl, nitro-phenylsulfenyl, tritylsulfenyl, o-nitrophenoxyacetyl and α-chlorobutyryl; (2) aromatic urethan type protecting groups such as benzyloxycarbonyl and substituted benzyloxycarbonyl, such as p-chlorobenzyloxycarbonyl, p-nitrobenzyl- carbonyl, p-bromobenzyloxycarbonyl, p-methoxybenzyloxycarbonyl, 1-(p-biphenylyl)-1-methylethoxycarbonyl, α, α-dimethyl-3,5-dimethoxybenzyloxycarbonyl and benzhydryloxycarbonyl, 9-fluorenylmethoxycarbonyl (Fmoc); (3) aliphatic urethan protecting groups such as tert-butyloxycarbonyl (Boc), diisopropylmethoxycarbonyl, isopropyloxycarbonyl, ethoxycarbonyl and allyloxycarbonyl; (4) cycloalkyl urethan type protecting groups such as cyclopentyloxycarbonyl, adamantylozycarbonyl and cyclohexyloxycarbonyl; (5) thio urethan type protecting groups such as phenylthiocarbonyl; (6) alkyl type protecting groups such as triphenylmethyl (trityl) and benzyl; and (7) trialkylsilane groups such as trimethylsilane. The preferred α-amino protecting group is tert-butyloxycarbonyl and Fmoc.

The selection of an appropriate coupling reagent is within the skill of the art. A particularly suitable coupling reagent where the amino acid to be added is Gln, Asn or Arg is N,N'-diisopropylcarbodiimide and 1-hydroxybenzotriazole. The use of these reagents prevents nitrile and lactam formation. Other coupling agents are (1) carbodiimides (e.g., N,N'-dicyclohexylcarbodiimide and N-ethyl-N'-(γ-dimethylaminopropylcarbodiimide); (2) cyanamides (e.g., N,N-dibenzylcyanamide); (3) ketenimines; (4) isoxazolium salts (e.g., N-ethyl-5-phenylisoxazolium-3'-sulfonate; (5) monocyclic nitrogen containing heterocyclic amides of aromatic character containing one through four nitrogens in the ring such as imidazolides, pyrazolides, and 1,2,4-triazolides. Specific heterocyclic amides that are useful include N,N'-carbonyldiimidazole and N,N-carbonyl-di-1,2,4-triazole; (6) alkoxylated acetylene (e.g., ethoxyacetylene); (7) reagents which form a mixed anhydride with the carboxyl moiety of the amino acid (e.g., ethylchloroformate and isobutylchloroformate) or the symmetrical anhydride of the amino acid to be coupled (e.g., (Boc-Ala)₂-O) and (8) nitrogen containing heterocyclic compounds having a hydroxy group on one ring nitrogen (e.g., N-hydroxyphthalimide, N-hydroxysuccinimide and 1-hydroxybenzotriazole). Other activating reagents and their use in peptide coupling are described by Kapoor, J. Pharm. Sci., 59, pp. 1-27 (1970). Applicants prefer the use of the symmetrical anhydride as a coupling reagent for all amino acids except Arg, Asn and Gln.

Each protected amino acid or amino acid sequence is introduced into the solid phase reactor in about a four-fold excess and the coupling is carried out in a medium of dimethylformamide: methylene chloride (1:1) or in dimethylformamide alone or preferably methylene chloride alone. In cases where incomplete coupling occurs, the coupling procedure is repeated before removal of the α-amino protecting group, prior to the coupling of the next amino acid in the solid phase reactor. The success of the coupling reaction at each stage of the synthesis is monitored by the ninhydrin reaction as described by E. Kaiser et al, Analyt. Biochem. 34, 595 (1970).

After the desired amino acid sequence has been obtained, the peptide is removed from the resin. This can be done by hydrolysis such as by treatment of the resin bound polypeptide with a solution of dimethyl sulfide, p-cresol and thiocresol in liquid hydrofluoric acid.

As is known in the art of solid phase peptide synthesis many of the amino acids bear functionalities requiring protection during the chain preparation. The use and selection of the appropriate protecting group is within the ability of those skilled in the art and will depend upon the amino acid to be protected and the presence of other protected amino acid residues on the peptide. The selection of such a side chain protecting group is critical in that it must be one which is not removed during cleavage of the protecting group of the α-amino moiety. For example, suitable side chain protecting groups for lysine are benzyloxycarbonyl and substituted benzyloxycarbonyl, said substituent being selected from halo (e.g., chloro, bromo, fluoro) and nitro (e.g., 2-chlorobenzyloxycarbonyl, p-nitrobenzyloxycarbonyl, 3,4-dichlorobenzyloxycarbonyl), tosyl, t-amyloxycarbonyl, t-butyloxycarbonyl and diisopropylmethoxycarbonyl. The alcoholic hydroxyl group of threonine and serine can be protected with an acetyl, benzoyl, tert-butyl, trityl, benzyl, 2,6-dichlorobenzyl or benzyloxycarbonyl group. The carboxylic group of Aspartic acid and Glutamic acid can be protected with a benzyl or cyclohexyl group. The preferred protecting group is benzyl.

These groups can be removed by procedures well known in the art. Typically protecting group removal is done after the peptide chain synthesis is complete but the protecting groups can be removed at any other appropriate time.

The ability of the peptide derivatives of formula 1 to act as antagonists of neuropeptide Y can be demonstrated by the ability of such peptides to compete with iodinated neuropeptide Y for receptors using the method of Lundberg et al. Eur. J. Pharmacol. 145:21-9 (1988). ¹²⁵I-Bolton-Hunter-neuropeptide Y (BHNPY; Amersham) binding was carried out in pig spleen crude membranes. Membranes from frozen spleen were prepared as described previously for tachykinin peptide binding studies (Buck S.H., et al., Science 266, 987-989 (1984)). An aliquot of membrane preparation (approximately 15 mg tissue) was incupated at room temperature for 2 hr in buffer (pH 7.4) containing the peptide analog, 130 mM NaCl, 2.7 mM KCl, 2 mM MgCl₂, 1.8 mM CaCl₂, 20 mM HEPES, 4 mg/ml BSA, 40 µg/ml bacitracin, 4 µg/ml leupeptin and 4 µg/mol chymostatin. BHNPY was included in a concentration of 0.1 nM and non-specific binding was determined by the inclusion of 1 µM pNPY. Samples were rapidly filtered over Whatman GF/C filters presoaked overnight in 0.5% histone (type II-AS; Sigma) and washed two times with ice-cold, plain HEPES-salt buffer (pH 7.4). IC₅₀ values for test peptides were calculated from 6 to 10 point competition curves. Utilizing this procedure the peptide derivative of Example 1 was found to have an IC₅₀ of < 300nM.

By virtue of the ability of the peptide derivatives of this invention to act as antagonists of neuropeptide Y, the compounds possess valuable pharmacologic properties such as hypotensive, anti-vasospasm, vasodilating and antihypertensive activity as well as depression of food intake desire and depression of luteinizing hormone secretion. Significant medical uses of the NPY antagonists of this invention are as antihypertensive agents, antianginal agents, antivasospasmotics, and as appetite depressants.

The dose of a peptide derivative of this invention required to antagonize neuropeptide Y and therefore produce a hypotensive, antihypertensive, vasodilator or other pharmacological or medical effect is from 0.2 mg/kg to 250 mg/kg of patient body weight per day depending on the patient, the severity of the thrombotic condition to be treated and the peptide derivative selected. The suitable dose for a particular patient can be readily determined. Preferably from 1 to 4 daily doses would be administered typically with from 5 mg to 100 mg of active compound per dose.

Hypotensive, antihypertensive, and vasodilator therapy is indicated for the treatment and prevention of a variety of conditions, particularly coronary artery and cerebrovascular disease such as hypertension and angina. Those experienced in this field are readily aware of the circumstances requiring such therapy. The term "patient" used herein is taken to mean mammals such as primates, including humans, sheep, horses, cattle, pigs, dogs, cats, rats and mice.

Although some of the peptide derivatives may survive passage through the gut following oral administration, applicants prefer non-oral administration, for example, subcutaneous, intravenous, intramuscular or intraperitoneal; administration by depot injection; by implant preparation; or by application to the mucous membranes, such as, that of the nose, throat and bronchial tubes, for example, in an aerosol can containing a peptide derivative of this invention in a spray or dry powder form.

For parenteral administration the compounds may be administered as injectable dosages of a solution or suspension of the compound in a physiologically acceptable diluent with a pharmaceutical carrier which can be a sterile liquid such as water and oils with or without the addition of a surfactant and other pharmaceutically acceptable adjuvants. Illustrative of oils which can be employed in these preparations are those of petroleum, animal, vegetable, or synthetic origin, for example, peanut oil, soybean oil, and mineral oil. In general, water, saline, aqueous dextrose and related sugar solutions, ethanol and glycols such as propylene glycol or polyethylene glycol are preferred liquid carriers, particularly for injectable solutions.

The compounds can be administered in the form of a depot injection or implant preparation which may be formulated in such a manner as to permit a sustained release of the active ingredient. The active ingredient can be compressed into pellets or small cylinders and implanted subcutaneously or intramuscularly as depot injections or implants. Implants may employ inert materials such as biodegradable polymers or synthetic silicones, for example, Silastic®, silicone rubber manufactured by the Dow-Corning Corporation, Midland, MI.

### EXAMPLES

This invention is illustrated by the following, nonlimiting examples.

### EXAMPLE 1

### PREPARATION OF

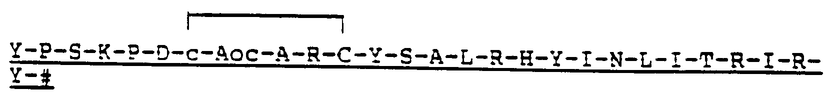

The title peptide derivative was synthesized on a 0.5 mmol scale by solid-phase methods on p-methylbenzhydrylamine resin (0.40 mmol/g; Peptides Intl.) using an ABI APPLIED BIOSYSTEMS®, Applied Biosystems Model 430-A Peptide Synthesizer. All residues were double coupled as the symmetrical anhydrides of she N^{α}-t-Boc-protected amino acids with the exception of Arg and Asn which were double coupled by the DCC/HOBT methodology. The side chain protection was as follows: Arg(Tos), Asp(Chx), Cys(pMeBzl), His(Tos), Ser(Bzl), Tyr(2-BrZ), Lys(2-Clz), Thr(Bzl). The peptides (0.25 mmol theory) were cleaved from the resin support and deprotected in liquid HF containing 5% anisole at -5°C for 40 min. After removal of the HF *in vacuo* the peptide was extracted from the resin with 30% acetic acid and water. The extract was diluted to 1 liter, the pH adjusted to between 8 and 9 with ammonium hydroxide and 0.01 N potassium ferricyanide was added until a yellow color persisted (approx. 25 ml). After stirring for 30 min, the pH was lowered to <5 with glacial acetic acid and the solution was stirred with 25 ml of settled AG 3-X4A resin (Bio-Rad®) for 2 hours. The solution was filtered from the resin and lyophilized. The peptidic material that remained was purified by preparative HPLC on a Dynamax® C-18 column (41.4 x 250 mm; Rainin) using acetonitrile in 0.1% trifluoroacetic acid as an eluant. The purity and identity of the peptide were assessed by analytical HPLC (Vydac® 218TP54 column, 4.6 cx 250 mm, 2.0 ml/min, t_{c} - 1.9 min, linear gradient of 15-40% acetonitrile in 0.1% TFA over 25 min), amino acid analysis (AAA)(6 N HCl containing 8% phenol; 106°C; 20 and 40 hr), and fast atom bombardment-mass spectrometry (FAB-MS)(M-Scan Ltd.).

AAA^{a}: B-1.96; T-1.03; S-1.62; P-1.88; A-1.96; I-2.84; L-2.14; Y-4.04; H-1.09; R-4.06.
^{a}6N HCl, 24 Hr, 106°C.
FAB-MS (M+H)⁺ 3311.2 ± 1 mu.

## Claims (Claims for the following Contracting State(s): AT, BE, CH, DE, FR, GB, IT, LI, LU, NL, SE)

1. A peptide derivative or the formula 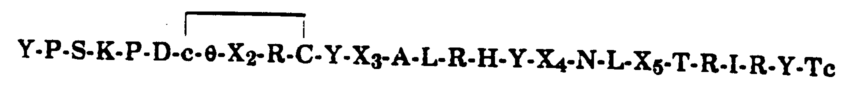 wherein
X₂ is S or A;
X₃ is S or A;
X₄ is L, I, M, Nle, or V;
X₅ Is L, I, M, Nle, or V;
Tc is OR' or NHR';
wherein R' is a hydrogen or a (C₁-C₄) alkyl group;
ϑ is a group of the structural formula
-NH-(CH₂)ₙ-CO₂-;
wherein n is an integer of from 1-11,
or a pharmaceutically acceptable salt thereof.

2. The peptide derivative of claim 1 wherein X₂ is A.

3. The peptide derivative of claim 1 or 2 wherein X₃ is S.

4. The peptide derivative of any one of claims 1 to 3 wherein X₄ is I.

5. The peptide derivative of any one of claims 1 to 4 wherein X₅ is I.

6. The peptide derivative of any one of claims 1 to 5 wherein Θ is Aoc.

7. The peptide derivative of any one of claims 1 to 6 wherein Tc is NH₂.

8. The peptide derivative of claim 1 which is

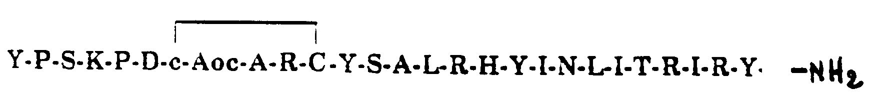

9. A process for preparing a peptide derivative of any one of claims 1 to 8 which comprises binding a suitably protected tyrosine to an activated resin support, subsequently binding the other alpha amino protected amino acids to the terminal amino group of the growing peptidic chain which has meanwhile been exposed by removing its amino protecting group, cleaving the protected peptide from the resin, removing any protecting groups, subjecting the linear peptide to an oxidative coupling, and finally isolating the cyclized peptide or a pharmaceutically acceptable salt thereof.

10. A peptide derivative or a pharmaceutically acceptable salt thereof according to any one of claims 1 to 8 or a mixture thereof for use as a pharmaceutically active compound.

11. A peptide derivative or a pharmaceutically acceptable salt thereof according to any one of claims 1 to 8 or a mixture thereof for the treatment of disorders requiring antagonizing a neuropeptide Y receptor.

12. A peptide derivative or a pharmaceutically acceptable salt thereof according to any one of claims 1 to 8 or a mixture thereof for the treatment of hypertension.

13. A peptide derivative or a pharmaceutically acceptable salt thereof according to any one of claims 1 to 8 or a mixture thereof for the treatment of angina.

14. A peptide derivative or a pharmaceutically acceptable salt thereof according to any one of claims 1 to 8 or a mixture thereof for the suppression of appetite.

15. A peptide derivative or a pharmaceutically acceptable salt thereof according to any one of claims 1 to 8 or a mixture thereof for the treatment of vasospasm.

16. A pharmaceutical composition containing a peptide derivative or a pharmaceutically acceptable salt thereof according to any one of claims 1 to 8 or a mixture thereof and optionally a pharmaceutically acceptable carrier and/or diluent.

17. The pharmaceutical composition according to claim 16 for the treatment of disorders requiring antagonizing a neuropeptide Y receptor, of hypertension, of angina, the suppression of appetite, or the treatment of vasospasm.

## Claims (Claims for the following Contracting State(s): ES)

1. A process for preparing a peptide derivative of the formula 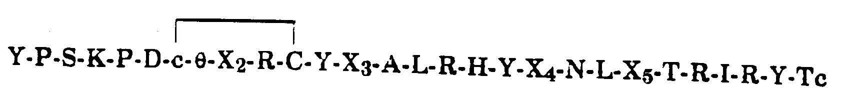 wherein
X₂ is S or A;
X₃ is S or A;
X₄ is L, I, M, Nle, or V;
X₅ is L, I, M, Nle, or V;
Tc is OR' or NHR';
wherein R' is a hydrogen or a (C₁-C₄) alkyl group;
Θ is a group of the structural formula
-NH-(CH₂)ₙ-CO₂-;
wherein n is an integer of from 1-11,
which comprises binding a suitably protected tyrosine to an activated resin support, subsequently binding the other alpha amino protected amino acids to the terminal amino group of the growing peptidic chain which has meanwhile been exposed by removing its amino protecting group, cleaving the protected peptide from the resin, removing any protecting groups, subjecting the linear peptide to an oxidative coupling, and finally isolating the cyclized peptide or a pharmaceutically acceptable salt thereof.

2. The process of claim 1 wherein X₂ is A.

3. The process of claim 1 or 2 wherein X₃ is S.

4. The process or any one of claims 1 to 3 wherein X₄ is I.

5. The process of any one of claims 1 to 4 wherein X₅ is I.

6. The process of any one of claims 1 to 5 wherein Θ is Aoc.

7. The process of any one of claims 1 to 6 wherein Tc is NH₂.

8. The process of claim 1 for preparing the compound

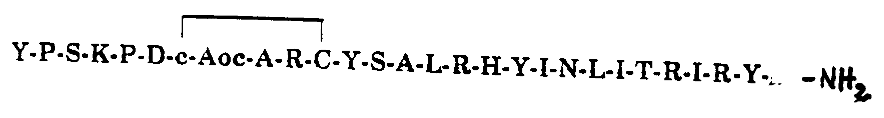

9. Use of a peptide derivative or a pharmaceutically acceptable salt thereof obtainable according to a process of any one of claims 1 to 8 or of a mixture thereof for the preparation of a pharmaceutical composition.

10. Use of a peptide derivative or a pharmaceutically acceptable salt thereof obtainable according to a process of any one of claims 1 to 8 or of a mixture thereof for the preparation of a pharmaceutical composition for the treatment of disorders requiring antagonizing a neuropeptide Y receptor.

11. Use of a peptide derivative or a pharmaceutically acceptable salt thereof obtainable according to a process of any one of claims 1 to 8 or of a mixture thereof for the preparation of a pharmaceutical composition for the treatment of hypertension.

12. Use of a peptide derivative or a pharmaceutically acceptable salt thereof obtainable according to a process of any one of claims 1 to 8 or of a mixture thereof for the preparation of a pharmaceutical composition for the treatment of angina.

13. Use of a peptide derivative or a pharmaceutically acceptable salt thereof obtainable according to a process of any one of claims 1 to 8 or of a mixture thereof for the preparation of a pharmaceutical composition for the suppression of appetite.

14. Use of a peptide derivative or a pharmaceutically acceptable salt thereof obtainable according to a process of any one of claims 1 to 8 or of a mixture thereof for the preparation of a pharmaceutical composition for the treatment of vasospasm.

## Claims (Claims for the following Contracting State(s): GR)

1. A peptide derivative of the formula 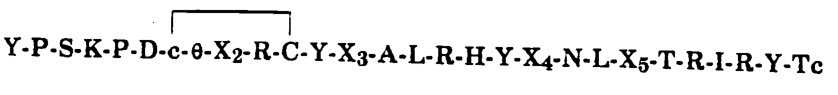 wherein
X₂ is S or A;
X₃ is S or A;
X₄ is L, I, M, Nle, or V;
X₅ is L, I, M, Nle, or V;
Tc is OR' or NHR';
wherein R' is a hydrogen or a (C₁-C₄) alkyl group;
Θ is a group of the structural formula
-NH-(CH₂)ₙ-CO₂-;
wherein n is an integer of from 1-11,
or a pharmaceutically acceptable salt thereof.

2. The peptide derivative of claim 1 wherein X₂ is A.

3. The peptide derivative of claim 1 or 2 wherein X₃ is S.

4. The peptide derivative of any one of claims 1 to 3 wherein X₄ is I.

5. The peptide derivative of any one of claims 1 to 4 wherein X₅ is I.

6. The peptide derivative of any one of claims 1 to 5 wherein Θ is Aoc.

7. The peptide derivative of any one of claims 1 to 6 wherein Tc is NH₂.

8. The peptide derivative or claim 1 which is

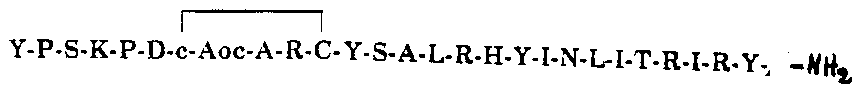

9. A process for preparing a peptide derivative of any one of claims 1 to 8 which comprises binding a suitably protected tyrosine to an activated resin support, subsequently binding the other alpha amino protected amino acids to the terminal amino group of the growing peptidic chain which has meanwhile been exposed by removing its amino protecting group, cleaving the protected peptide from the resin, removing any protecting groups, subjecting the linear peptide to an oxidative coupling, and finally isolating the cyclized peptide or a pharmaceutically acceptable salt thereof.

10. A peptide derivative or a pharmaceutically acceptable salt thereof according to any one of claims 1 to 8 or a mixture thereof for use as a pharmaceutically active compound.

11. A peptide derivative or a pharmaceutically acceptable salt thereof according to any one of claims 1 to 8 or a mixture thereof for the treatment of disorders requiring antagonizing a neuropeptide Y receptor.

12. A peptide derivative or a pharmaceutically acceptable salt thereof according to any one of claims 1 to 8 or a mixture thereof for the treatment of hypertension.

13. A peptide derivative or a pharmaceutically acceptable salt thereof according to any one of claims 1 to 8 or a mixture thereof for the treatment of angina.

14. A peptide derivative or a pharmaceutically acceptable salt thereof according to any one of claims 1 to 8 or a mixture thereof for the suppression of appetite.

15. A peptide derivative or a pharmaceutically acceptable salt thereof according to any one of claims 1 to 8 or a mixture thereof for the treatment of vasospasm.

## Patentansprüche (Patentansprüche für folgende(n) Vertragsstaat(en): AT, BE, CH, DE, FR, GB, IT, LI, LU, NL, SE)

1. Peptidderivat der Formel 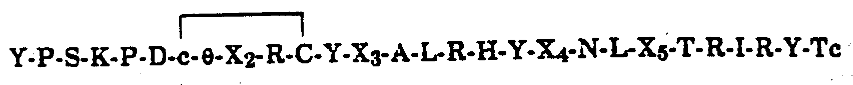 in der
X₂ S oder A bedeutet;
X₃ S oder A bedeutet,
X₄ L, I, M, Nle oder V bedeutet;
X₅ L, I, M, Nle oder V bedeutet;
Tc OR' oder NHR' bedeutet,
wobei R' ein Wasserstoffatom oder einen (C₁-C₄)-Alkylrest darstellt;
Θ eine Gruppe der Strukturformel
-NH-(CH₂)ₙ-CO₂-
bedeutet,
wobei n eine ganze Zahl im Wert von 1 bis 11 darstellt, oder ein pharmazeutisch verträgliches Salz davon.

2. Peptidderivat nach Anspruch 1, wobei X₂ A bedeutet.

3. Peptidderivat nach Anspruch 1 oder 2, wobei X₃ S bedeutet.

4. Peptidderivat nach einem der Ansprüche 1 bis 3, wobei X₄ I bedeutet.

5. Peptidderivat nach einem der Ansprüche 1 bis 4, wobei X₅ I bedeutet.

6. Peptidderivat nach einem der Ansprüche 1 bis 5, wobei Θ Aoc bedeutet.

7. Peptidderivat nach einem der Ansprüche 1 bis 6, wobei Tc NH₂ bedeutet.

8. Peptidderivat nach Anspruch 1, nämlich

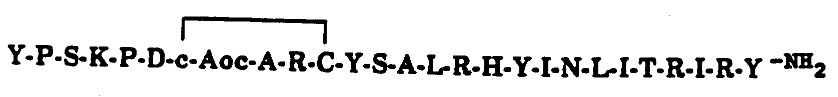

9. Verfahren zur Herstellung eines Peptidderivats nach einem der Ansprüche 1 bis 8, umfassend das Binden eines geeignet geschützten Tyrosins an einen aktivierten Harzträger, anschließendes Binden der anderen α-aminogeschützten Aminosäuren an die terminale Aminogruppe der wachsenden Peptidkette, die mittlerweile durch Entfernung ihrer Aminoschutzgruppe freigelegt wurde, Abspaltung des geschützten Peptids vom Harz, Entfernung vorhandener Schutzgruppen, Durchführung einer oxidativen Kupplung des linearen Peptids und schließlich Isolierung des cyclisierten Peptids oder eines pharmazeutisch verträglichen Salzes davon.

10. Peptidderivat oder ein pharmazeutisch verträgliches Salz davon nach einem der Ansprüche 1 bis 8 oder ein Gemisch davon zur Verwendung als einen pharmazeutischen Wirkstoff.

11. Peptidderivat oder ein pharmazeutisch verträgliches Salz davon nach einem der Ansprüche 1 bis 8 oder ein Gemisch davon für die Behandlung von Erkrankungen, die die Hemmung eines Neuropeptid Y-Rezeptors erfordern.

12. Peptidderivat oder ein pharmazeutisch verträgliches Salz davon nach einem der Ansprüche 1 bis 8 oder ein Gemisch davon für die Behandlung von Bluthochdruck.

13. Peptidderivat oder ein pharmazeutisch verträgliches Salz davon nach einem der Ansprüche 1 bis 8 oder ein Gemisch davon für die Behandlung von Angina.

14. Peptidderivat oder ein pharmazeutisch verträgliches Salz davon nach einem der Ansprüche 1 bis 8 oder ein Gemisch davon für die Appetithemmung.

15. Peptidderivat oder ein pharmazeutisch verträgliches Salz davon nach einem der Ansprüche 1 bis 8 oder ein Gemisch davon für die Behandlung von Vasospasmus.

16. Arzneimittel, enthaltend ein Peptidderivat oder ein pharmazeutisch verträgliches Salz davon nach einem der Ansprüche 1 bis 8 oder ein Gemisch davon und gegebenenfalls einen pharmazeutisch verträglichen Träger und/oder ein Verdünnungsmittel.

17. Arzneimittel nach Anspruch 16 für die Behandlung von Erkrankungen, die die Hemmung eines Neuropeptid Y-Rezeptors erfordern, von Bluthochdruck, Angina, für die Appetithemmung oder für die Behandlung von Vasospasmus.

## Patentansprüche (Patentansprüche für folgende(n) Vertragsstaat(en): ES)

1. Verfahren zur Herstellung eines Peptidderivats der Formel 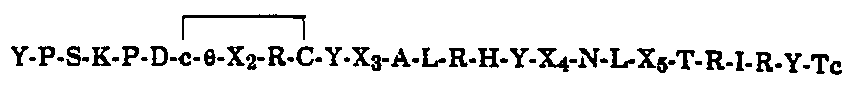 in der
X₂ S oder A bedeutet;
X₃ S oder A bedeutet,
X₄ L, I, M, Nle oder V bedeutet;
X₅ L, I, M, Nle oder V bedeutet;
Tc OR' oder NHR' bedeutet,
wobei R' ein Wasserstoffatom oder einen (C₁-C₄)-Alkylrest darstellt;
Θ eine Gruppe der Strukturformel
-NH-(CH₂)ₙ-CO₂-
bedeutet,
wobei n eine ganze Zahl im Wert von 1 bis 11 darstellt, umfassend das Binden eines geeignet geschützten Tyrosins an einen aktivierten Harzträger, anschließendes Binden der anderen α-aminogeschützten Aminosäuren an die terminale Aminogruppe der wachsenden Peptidkette, die mittlerweile durch Entfernung ihrer Aminoschutzgruppe freigelegt wurde, Abspaltung des geschützten Peptids vom Harz, Entfernung vorhandener Schutzgruppen, Durchführung einer oxidativen Kupplung des linearen Peptids und schließlich Isolierung des cyclisierten Peptids oder eines pharmazeutisch verträglichen Salzes davon.

2. Verfahren nach Anspruch 1, wobei X₂ A bedeutet.

3. Verfahren nach Anspruch 1 oder 2, wobei X₃ S bedeutet.

4. Verfahren nach einem der Ansprüche 1 bis 3, wobei X₄ I bedeutet.

5. Verfahren nach einem der Ansprüche 1 bis 4, wobei X₅ I bedeutet.

6. Verfahren nach einem der Ansprüche 1 bis 5, wobei Θ Aoc bedeutet.

7. Verfahren nach einem der Ansprüche 1 bis 6, wobei Tc NH₂ bedeutet.

8. Verfahren nach Anspruch 1 zur Herstellung der Verbindung

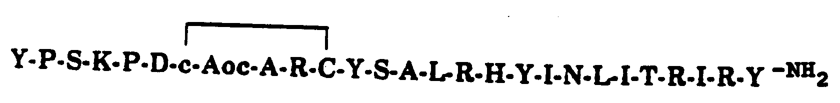

9. Verwendung eines Peptidderivats oder eines pharmazeutisch verträglichen Salzes davon, erhältlich gemäß dem Verfahren nach einem der Ansprüche 1 bis 8, oder eines Gemisches davon für die Herstellung eines Arzneimittels.

10. Verwendung eines Peptidderivats oder eines pharmazeutisch verträglichen Salzes davon, erhältlich gemäß dem Verfahren nach einem der Ansprüche 1 bis 8, oder eines Gemisches davon für die Herstellung eines Arzneimittels für die Behandlung von Erkrankungen, die die Hemmung eines Neuropeptid Y-Rezeptors erfordern.

11. Verwendung eines Peptidderivats oder eines pharmazeutisch verträglichen Salzes davon, erhältlich gemäß einem Verfahren nach einem der Ansprüche 1 bis 8, oder eines Gemisches davon für die Herstellung eines Arzneimittels für die Behandlung von Bluthochdruck.

12. Verwendung eines Peptidderivats oder eines pharmazeutisch verträglichen Salzes davon, erhältlich gemäß einem Verfahren nach einem der Ansprüche 1 bis 8, oder eines Gemisches davon für die Herstellung eines Arzneimittels für die Behandlung von Angina.

13. Verwendung eines Peptidderivats oder eines pharmazeutisch verträglichen Salzes davon, erhältlich gemäß einem Verfahren nach einem der Ansprüche 1 bis 8, oder eines Gemisches davon für die Herstellung eines Arzneimittels für die Appetithemmung.

14. Verwendung eines Peptidderivats oder eines pharmazeutisch verträglichen Salzes davon, erhältlich gemäß einem Verfahren nach einem der Ansprüche 1 bis 8, oder eines Gemisches davon für die Herstellung eines Arzneimittels für die Behandlung von Vasospasmus.

## Patentansprüche (Patentansprüche für folgende(n) Vertragsstaat(en): GR)

1. Peptidderivat der Formel 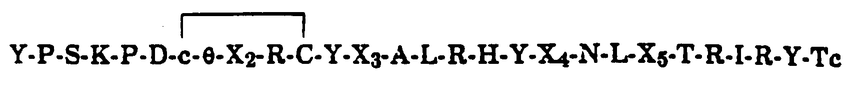 in der
X₂ S oder A bedeutet;
X₃ S oder A bedeutet,
X₄ L, I, M, Nle oder V bedeutet;
X₅ L, I, M, Nle oder V bedeutet;
Tc OR' oder NHR' bedeutet,
wobei R' ein Wasserstoffatom oder einen (C₁-C₄)-Alkylrest darstellt;
Θ eine Gruppe der Strukturformel
-NH-(CH₂)ₙ-CO₂-
bedeutet,
wobei n eine ganze Zahl im Wert von 1 bis 11 darstellt, oder ein pharmazeutisch verträgliches Salz davon.

2. Peptidderivat nach Anspruch 1, wobei X₂ A bedeutet.

3. Peptidderivat nach Anspruch 1 oder 2, wobei X₃ S bedeutet.

4. Peptidderivat nach einem der Ansprüche 1 bis 3, wobei X₄ I bedeutet.

5. Peptidderivat nach einem der Ansprüche 1 bis 4, wobei X₅ I bedeutet.

6. Peptidderivat nach einem der Ansprüche 1 bis 5, wobei Θ Aoc bedeutet.

7. Peptidderivat nach einem der Ansprüche 1 bis 6, wobei Tc NH₂ bedeutet.

8. Peptidderivat nach Anspruch 1, nämlich

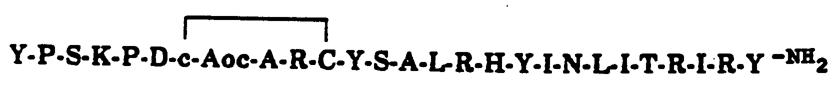

9. Verfahren zur Herstellung eines Peptidderivats nach einem der Ansprüche 1 bis 8, umfassend das Binden eines geeignet geschützten Tyrosins an einen aktivierten Harzträger, anschließendes Binden der anderen α-aminogeschützten Aminosäuren an die terminale Aminogruppe der wachsenden Peptidkette, die mittlerweile durch Entfernung ihrer Aminoschutzgruppe freigelegt wurde, Abspaltung des geschützten Peptids vom Harz, Entfernung vorhandener Schutzgruppen, Durchführung einer oxidativen Kupplung des linearen Peptids und schließlich Isolierung des cyclisierten Peptids oder eines pharmazeutisch verträglichen Salzes davon.

10. Peptidderivat oder ein pharmazeutisch verträgliches Salz davon nach einem der Ansprüche 1 bis 8 oder ein Gemisch davon zur Verwendung als einen pharmazeutischen Wirkstoff.

11. Peptidderivat oder ein pharmazeutisch verträgliches Salz davon nach einem der Ansprüche 1 bis 8 oder ein Gemisch davon für die Behandlung von Erkrankungen, die die Hemmung eines Neuropeptid Y-Rezeptors erfordern.

12. Peptidderivat oder ein pharmazeutisch verträgliches Salz davon nach einem der Ansprüche 1 bis 8 oder ein Gemisch davon für die Behandlung von Bluthochdruck.

13. Peptidderivat oder ein pharmazeutisch verträgliches Salz davon nach einem der Ansprüche 1 bis 8 oder ein Gemisch davon für die Behandlung von Angina.

14. Peptidderivat oder ein pharmazeutisch verträgliches Salz davon nach einem der Ansprüche 1 bis 8 oder ein Gemisch davon für die Appetithemmung.

15. Peptidderivat oder ein pharmazeutisch verträgliches Salz davon nach einem der Ansprüche 1 bis 8 oder ein Gemisch davon für die Behandlung von Vasospasmus.

## Revendications (Revendications pour l'(les) Etat(s) contractant(s) suivant(s): AT, BE, CH, DE, FR, GB, IT, LI, LU, NL, SE)

1. Dérivé de peptide de formule 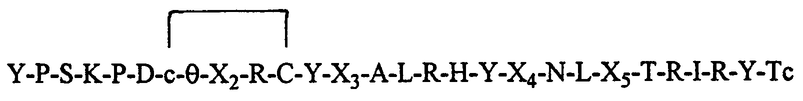 dans laquelle
X₂ est S ou A;
X₃ est S ou A;
X₄ est L, I, M, Nle, ou V;
X₅ est L, I, M, Nle, ou V;
Tc est OR' ou NHR' ;
avec R' étant un hydrogène ou un coupe alkyle ayant de 1 à 4 atomes de carbone;
ϑ est un groupe de formule structurale
-NH-(CH₂)ₙ-CO₂-;
dans laquelle n est un entier de 1 à 11,
ou un de ses sels pharmaceutiquement acceptables.

2. Dérivé de peptide de la revendication 1, dans lequel X₂ est A.

3. Dérivé de peptide de la revendication 1 ou 2, dans lequel X₃ est S.

4. Dérivé de peptide de l'une quelconque des revendications 1 à 3, dans lequel X₄ est I.

5. Dérivé de peptide de l'une quelconque des revendications 1 à 4, dans lequel X₅ est I.

6. Dérivé de peptide de l'une quelconque des revendications 1 à 5, dans lequel ϑ est Aoc.

7. Dérivé de peptide de l'une quelconque des revendications 1 à 6, dans lequel Tc est NH₂.

8. Dérivé de peptide de la revendication 1, qui est

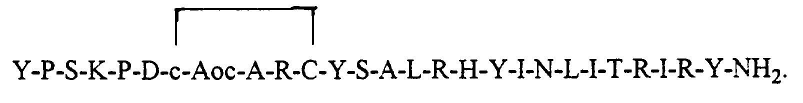

9. Procédé pour préparer un dérivé de peptide de l'une quelconque des revendications 1 à 8 qui comprend la fixation d'une tyrosine convenablement protégée sur un support de résine activée, puis la fixation des autres amino acides protégés sur l'amino en alpha au groupe amino terminal de la chaine peptidique en cours de formation, groupe qui a entre-temps été libéré par élimination de son groupe amino-protecteur, le clivage du peptide protégé de la résine, l'élimination de tous les groupes protecteurs, la soumission du peptide linéaire à un couplage oxydant, et enfin l'isolement du peptide cyclisé ou d'un de ses sels pharmaceutiquement acceptables.

10. Dérivé de peptide ou un de ses sels pharmaceutiquement acceptables selon une quelconque des revendications 1 à 8 ou un de leurs mélanges pour leur utilisation en tant que composé pharmaceutiquement actif.

11. Dérivé de peptide ou un de ses sels pharmaceutiquement acceptables selon une quelconque des revendications 1 à 8 ou un de leurs mélanges pour le traitement des troubles nécessitant d'antagoniser un récepteur de neuropeptide Y.

12. Dérivé de peptide ou un de ses sels pharmaceutiquement acceptables selon une quelconque des revendications 1 à 8 ou un de leurs mélanges pour le traitement de l'hypertension.

13. Dérivé de peptide ou un de ses sels pharmaceutiquement acceptables selon une quelconque des revendications 1 à 8 ou un de leurs mélanges pour le traitement de l'insuffisance coronarienne.

14. Dérivé de peptide ou un de ses sels pharmaceutiquement acceptables selon une quelconque des revendications 1 à 8 ou un de leurs mélanges pour la suppression de l'appétit.

15. Dérivé de peptide ou un de ses sels pharmaceutiquement acceptables selon une quelconque des revendications 1 à 8 ou un de leurs mélanges pour le traitement des spasmes vasculaires.

16. Composition pharmaceutique contenant un dérivé de peptide ou un de ses sels pharmaceutiquement acceptables selon une quelconque des revendications 1 à 8 ou un de leurs mélanges et optionnellement un vecteur et/ou diluant pharmaceutiquement acceptable.

17. Composition pharmaceutique selon la revendication 16 pour le traitement des troubles nécessitant d'antagoniser un récepteur de neuropeptide Y, pour le traitement de l'hypertension, de l'insuffisance coronarienne, pour supprimer l'appétit ou pour le traitement des spasmes vasculaires.

## Revendications (Revendications pour l'(les) Etat(s) contractant(s) suivant(s): ES)

1. Procédé pour préparer un dérivé de peptide de formule 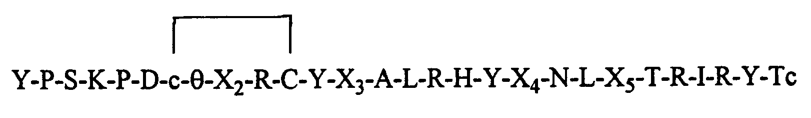 dans laquelle
X₂ est S ou A;
X₃ est S ou A;
X₄ est L, I, M, Nle, ou V;
X₅ est L, I, M, Nle, ou V;
Tc est OR' ou NHR';
avec R' étant un hydrogène ou un groupe alkyle ayant de 1 à 4 atomes de carbone;
ϑ est a groupe de formule structurale
-NH-(CH₂)ₙ-CO₂-;
dans laquelle n est un entier de 1 à 11,
qui comprend la fixation d'une tyrosine convenablement protégée sur un support de résine activée, puis la fixation des autres amino acides protégés sur l'amino en alpha au groupe amino terminal de la chaine peptidique en cours de formation, groupe qui a entre-temps été libéré par élimination de son groupe amino-protecteur, le clivage du peptide protégé de la résine, l'élimination de tous les groupes protecteurs, la soumission du peptide linéaire à un couplage oxydant, et enfin l'isolement du peptide cyclisé ou d'un de ses sels pharmaceutiquement acceptables.

2. Procédé de la revendication 1, dans laquelle X₂ est A.

3. Procédé de la revendication 1 ou 2, dans laquelle X₃ est S.

4. Procédé de l'une quelconque des revendications 1 à 3, dans laquelle X₄ est I.

5. Procédé de l'une quelconque des revendications 1 à 4, dans laquelle X₅ est I.

6. Procédé de l'une quelconque des revendications 1 à 5, dans laquelle ϑ est Aoc.

7. Procédé de l'une quelconque des revendications 1 à 6, dans laquelle Tc est NH₂.

8. Procédé de la revendication 1, pour préparer le composé

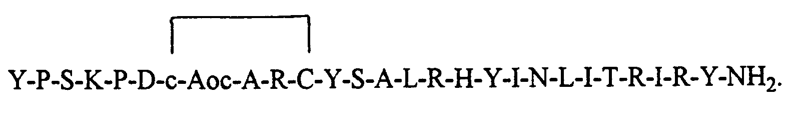

9. Utilisation d'un dérivé de peptide ou un de ses sels pharmaceutiquement acceptables obtenus selon un procédé d'une quelconque des revendications 1 à 8 ou un de leurs mélanges pour la préparation d'une composition pharmaceutique.

10. Utilisation d'un dérivé de peptide ou un de ses sels pharmaceutiquement acceptables obtenus selon un procédé d'une quelconque des revendications 1 à 8 ou un de leurs mélanges pour la préparation d'une composition pharmaceutique pour le traitement des troubles nécessitant d'antagoniser un récepteur de neuropeptide Y.

11. Utilisation d'un dérivé de peptide ou un de ses sels pharmaceutiquement acceptables obtenus selon un procédé d'une quelconque des revendications 1 à 8 ou un de leurs mélanges pour la préparation d'une composition pharmaceutique pour le traitement de l'hypertension.

12. Utilisation d'un dérivé de peptide ou un de ses sels pharmaceutiquement acceptables obtenus selon un procédé d'une quelconque des revendications 1 à 8 ou un de leurs mélanges pour la préparation d'une composition pharmaceutique pour le traitement de l'insuffissance coronarienne.

13. Utilisation d'un dérivé de peptide ou un de ses sels pharmaceutiquement acceptables obtenus selon un procédé d'une quelconque des revendications 1 à 8 ou un de leurs mélanges pour la préparation d'une composition pharmaceutique pour la suppression de l'appétit.

14. Utilisation d'un dérivé de peptide ou un de ses sels pharmaceutiquement acceptables obtenus selon un procédé d'une quelconque des revendications 1 à 8 ou un de leurs mélanges pour la préparation d'une composition pharmaceutique pour le traitement des spasmes vasculaires.

## Revendications (Revendications pour l'(les) Etat(s) contractant(s) suivant(s): GR)

1. Dérivé de peptide de formule 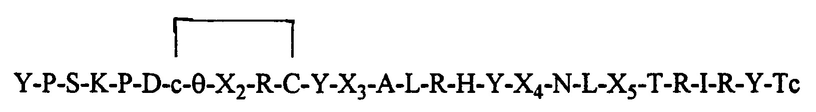 dans laquelle
X₂ est S ou A;
X₃ est S ou A;
X₄ est L, I, M, Nle, ou V;
X₅ est L, I, M, Nle, ou V;
Tc est OR' ou NHR';
avec R' étant un hydrogène ou un groupe alkyle ayant de 1 à 4 atomes de carbone;
ϑ est un groupe de formule structurale
-NH-(CH₂)ₙ-CO₂-;
dans laquelle n est un entier de 1 à 11,
ou un de ses sels pharmaceutiquement acceptables.

2. Dérivé de peptide de la revendication 1, dans lequel X₂ est A.

3. Dérivé de peptide de la revendication 1 ou 2, dans lequel X₃ est S.

4. Dérivé de peptide de l'une quelconque des revendications 1 à 3, dans lequel X₄ est I.

5. Dérivé de peptide de l'une quelconque des revendications 1 à 4, dans lequel X₅ est I.

6. Dérivé de peptide de l'une quelconque des revendications 1 à 5, dans lequel ϑ est Aoc.

7. Dérivé de peptide de l'une quelconque des revendications 1 à 6, dans lequel Tc est NH₂.

8. Dérivé de peptide de la revendication 1, qui est

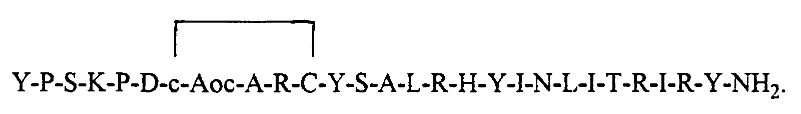

9. Procédé pour préparer un dérivé de peptide de l'une quelconque des revendications 1 à 8 qui comprend la fixation d'une tyrosine convenablement protégée sur un support de résine activée, puis la fixation des autres amino acides protégés sur l'amino en alpha au groupe amino terminal de la chaine peptidique en cours de formation, groupe qui a entre-temps été libéré par élimination de son groupe amino-protecteur, le clivage du peptide protégé de la résine, l'élimination de tous les groupes protecteurs, la soumission du peptide linéaire à un couplage oxydant, et enfin l'isolement du peptide cyclisé ou d'un de ses sels pharmaceutiquement acceptables.

10. Dérivé de peptide ou un de ses sels pharmaceutiquement acceptables selon une quelconque des revendications 1 à 8 ou un de leurs mélanges pour leur utilisation en tant que composé pharmaceutiquement actif.

11. Dérivé de peptide ou un de ses sels pharmaceutiquement acceptables selon une quelconque des revendications 1 à 8 ou un de leurs mélanges pour le traitement des troubles nécessitant d'antagoniser un récepteur de neuropeptide Y.

12. Dérivé de peptide ou un de ses sels pharmaceutiquement acceptables selon une quelconque des revendications 1 à 8 ou un de leurs mélanges pour le traitement de l'hypertension.

13. Dérivé de peptide ou un de ses sels pharmaceutiquement acceptables selon une quelconque des revendications 1 à 8 ou un de leurs mélanges pour le traitement de l'insuffisance coronarienne.

14. Dérivé de peptide ou un de ses sels pharmaceutiquement acceptables selon une quelconque des revendications 1 à 8 ou un de leurs mélanges pour la suppression de l'appétit.

15. Dérivé de peptide ou un de ses sels pharmaceutiquement acceptables selon une quelconque des revendications 1 à 8 ou un de leurs mélanges pour le traitement des spasmes vasculaires.
